Europäisches Patentamt

European Patent Office

Office européen des brevets

(11) Publication number: **0 336 481 B1**

## EUROPEAN PATENT SPECIFICATION

(45) Date of publication of patent specification: **18.05.94**    (51) Int. Cl.5: **G01N 33/535**, C12Q 1/28

(21) Application number: **89200761.8**

(22) Date of filing: **24.03.89**

(54) **Method for detecting a specific binding substance in blood and also a test kit for carrying out said method.**

(30) Priority: **06.04.88 NL 8800874**

(43) Date of publication of application:
**11.10.89 Bulletin 89/41**

(45) Publication of the grant of the patent:
**18.05.94 Bulletin 94/20**

(84) Designated Contracting States:
**AT BE CH DE ES FR GB GR IT LI NL SE**

(56) References cited:
**EP-A- 0 121 317
EP-A- 0 177 244
WO-A-86/05207
DE-A- 3 506 365**

**ANALYTICAL BIOCHEMISTRY, vol. 76, 1976, pages 184-191, Academic Press Inc., New York, US; R.L. HEATH et al.: "A new sensitive assay for the measurement of hydroperoxides"**

**J. CLIN. CHEM. CLIN. BIOCHEM., vol. 19, 1981, pages 435-439, Walter de Gruyter & Co., Berlin, DE; B. PORSTMANN et al.: "Comparison of chromogens for the determination of horseradish peroxidase as a marker in enzyme immunoassay"**

(73) Proprietor: **Akzo Nobel N.V.
Velperweg 76
NL-6824 BM Arnhem(NL)**

(72) Inventor: **Gribnau, Thomas Cornelis Josephus
Langven 2a
NL-5384 PT Heesch(NL)**
Inventor: **Kuijpers, Leonardus Paulus Clemens
Singel 40
NL-5395 TC Teeffelen(NL)**
Inventor: **Goossens, Petrus Adrianus Leonardus
Lisztgaarde 218
NL-5344 EM Oss(NL)**

(74) Representative: **Hermans, Franciscus G.M. et al
Patent Department
AKZO NOBEL N.V.
Pharma Division
P.O. Box 20
NL-5340 BH Oss (NL)**

EP 0 336 481 B1

**Description**

The invention relates to a method for quantitatively and/or qualitatively detecting a specific binding substance in blood or a derived fraction thereof using the binding affinity between the specific binding substance and its co-reactant. Moreover a reaction component with peroxidase activity has been used by incubating a reaction mixture containing the specific binding substance and its co-reactant and by which the peroxidase activity in said reaction mixture or a fraction derived therefrom has been determined. The peroxidase activity is a measure of the quantity and/or presence of the specific binding substance. Also a test kit for carrying out said method is part of the invention. Such a method to demonstrate a peroxidase activity has already been described in Dutch Patent 154,600.

As carrier material in said method has often been used the wall of a small well in a microtitration plate to which antibodies are bonded. The blood to be investigated or a fraction derived therefrom, such as serum, is introduced into the small wells of the microtitration plate. The bonded antibody is able to react with the antigen present in the blood or a derived fraction thereof, as a result of which said antigen is bonded. In order to be able to detect whether an immunochemical reaction has taken place, subsequently one can use marked antibodies which are directed against the antigen to be determined. Said marked antibodies will react with the antibody-antigen complex formed already on the solid phase. The marked antibodies consist of antibodies to which an enzyme is bonded, preferably a peroxidase-enzyme. In the next step, a colourless solution (substrate + chromogen) is added. The enzyme is able to convert this solution into a coloured compound. The intensity of the colour depends on the quantity of enzyme which is proportional to the quantity of bound antigen. Said colour can be altered by adding a stop reagent. The method thus described of determining an antigen in blood or a derived fraction thereof is a method of the sandwich type. Other immunochemical methods, such as an inhibition reaction and a competition reaction, can also be used. As suitable enzymes one can use, inter alia, alkaline phosphatase, urease and a previously mentioned peroxidase, preferably Horse Radish Peroxidase (HRP). The HRP catalyses the conversion of the substrate, a peroxide compound, in water. The electrons needed for this reaction may be supplied by chromogenic substances such as tetramethylbenzidine (TMB). The TMB is converted as a result into coloured complexes, by which the enzyme action is demonstrated. The colour formed can either be detected visually or the enzyme action can be determined in an analytically suitable manner.

In examining blood for the presence of antigens or antibodies, it is usual that the serum containing the antigens or antibodies to be determined has been separated from the blood cells prior to the determination.

In practice it emerges that an undesired, increased colour intensity is often produced, in particular in carrying out an enzyme immunoassay (EIA) when a peroxidase being used as enzyme. This phenomenon is also described as partial coloration.

It will be clear that this phenomenon of partial coloration will lead to undesired results and conclusions. In this connection one can mention so-called AIDS antibody tests. In these tests sera of blood donors are tested routinely in an enzyme immunoassay for the presence of antibodies directed against the AIDS virus. The present invention is therefore characterized in that the determination of the peroxidase activity is carried out in the presence of a quaternary ammonium salt.

As quaternary ammonium salts one can use for example dodecyltrimethylammonium bromide, hexadecyltrimethylammonium bromide, tetradecyltrimethylammonium bromide, alkyldimethylbenzylammonium chloride, benzethonium chloride, benzyldimethylhexadecylammonium chloride, cetyldimethylethylammonium bromide, cetylpyridinium bromide, cetylpyridinium chloride, dimethyldioctadecylammonium bromide and methylbenzethonium chloride. As preference for suitable quaternary ammonium salts, use is made of dodecyltrimethylammonium bromide (DTAB), hexadecyltrimethylammonium bromide (CTAB) or alkyldimethylbenzylammonium chloride (benzalkonium chloride).

It is surprising that the peroxidase activity can be determined by adding said quaternary ammonium salts, while the partial coloration being significantly suppressed. If desired, whole blood can be used diluted or undiluted as a sample, the red blood cells being removed as well as possible before determining the peroxidase activity. Quaternary ammonium salt can be added to the test in the form of a solution containing the quaternary ammonium salt or as a solid compound.

This addition of the quaternary ammonium salt to the test may be carried out either together with the substrate, or together with the chromogen, or together with a substrate/chromogen mixture.

The invention also relates to a test kit which contains the components such as a peroxidase-enzyme-conjugate and a quaternary ammonium salt for carrying out the method according to the invention.

The method according to the invention for quantitatively and/or qualitatively detecting a specific binding substance in blood or a derived fraction thereof can be carried out in any reaction in which the binding of the specifically binding substance with its co-reactant is determined by using a reaction component having

EP 0 336 481 B1

peroxidase activity. The determination of the peroxidase activity is a measure of the quantity and/or presence of the specifically binding substance.

Thus, one can use, for example, the previously mentioned sandwich- but also an inhibition- or competition reaction.

A "specifically binding substance" may be understood to mean for example a hapten, an antigen or a fragment thereof, an antibody or a fragment thereof or protein A, DNA or RNA.

"Its co-reactant" may be understood to mean for example, a hapten, an antigen or a fragment thereof, an antibody or a fragment thereof or protein A, DNA or RNA, said co-reactant having a binding affinity for the specifically binding substance. This co-reactant may optionally be bound to carrier material.

A "reaction component" may be understood to mean for example, a hapten, an antigen or a fragment thereof, an antibody or a fragment thereof or protein A, DNA or RNA, by which said reaction component also has peroxidase activity, which component does not need to be bound to a solid carrier and which component can be used in solid form, in freeze-dried form or in solution.

If the antigen is detected in the method according to the invention with the aid of a sandwich reaction, said antigen can be incubated with a carrier material to which antibodies which are directed against the antigen to be determined have already been bound. As carrier one may use for example test tubes, microtitration plates, rods, beads or discs made of, for example, glass or plastic. The detection of whether complex formation has occurred between the antibody bound to the solid carrier and the antigen to be determined is carried out by subsequently adding a second antibody provided with an enzyme.

The enzyme is selected, inter alia, on the basis of reactivity and stability. Enzymes such as alkaline phosphatase, urease, glucose oxidase, galactose oxidase and peroxidase enjoy preference, in particular the group of the peroxidases. Said enzymes are capable of catalysing a conversion of a peroxide-substrate, in which coloured components are involved. Within this group, HRP enzyme has a pronounced preference owing, primarily, to good stability and low cost price. The electron-supplying substances also needed for said enzyme reaction may, for example, be chromogens. Suitable chromogens are, for example, tetramethylbenzidine, o-phenylenediamine, 5-aminosalicylic acid, 2,2′-azino-di-[3-ethylbenzothiazoline-6-sulphonate], the preference being for tetramethylbenzidine (TMB). Said TMB is converted during the reaction into coloured complexes, as a result of which the enzyme action is detected, and this provides a qualitative and/or quantitative measure of the antigen to be determined.

The invention is explained further with reference to the following example.

Example

Detection of HBsAq (Hepatitis B surface antigen) in serum.

A serum pool which was negative for HBsAg was examined.

A. Reagents

1. Coated microtitration plates
The small wells of polystyrene microtitration plates were coated on the inside with a monoclonal antibody directed against HBsAg by a method known in immunochemistry.
2. Conjugate
A conjugate of antibodies directed against HBsAg and horse radish peroxidase (HRP) was prepared according to a method which is conventional in immunochemistry.
3. Substrate/chromogen
As substrate, use was made of a urea peroxidase solution, while as chromogen, use was made of a TMB solution (tetramethylbenzidine dissolved in dimethyl sulphoxide). The substrate/chromogen solution was divided into two equal parts. To one part a quaternary ammonium salt (CTAB) was added in a concentration of 0.2 g/l (group A), while no quaternary ammonium salt was introduced into the other part (group B).

B. Performance of the test The coated microtitration plates were incubated for 30 minutes at 50°C with 0.1 ml of the serum to be examined in the respective small wells.

Subsequently the small wells were drained and washed 4 times with a washing buffer (PBS/Tween[R]) and drained again.

3

0.1 ml of the conjugate was subsequently introduced into each of the small wells. After an incubation for 30 minutes at 50°C, the small wells were again drained, washed and drained.

Subsequently the microtitration plates were divided into two groups: group A and group B. The small wells in the plates belonging to group A then received 0.1 ml of substrate/chromogen mixture together with the CTAB. The small wells in the plates belonging to group B received the substrate/chromogen mixture to which no CTAB had been added.

After incubating for 30 minutes at 25°C, the enzyme reaction was stopped by adding 0.1 ml of sulphuric acid solution (2 M) to each small well.

C. <u>Results</u> The results obtained from both groups are shown in the table.

<div align="center">

<u>Table</u>

</div>

|  | CTAB added (group A) | CTAB not added (group B) |
|---|---|---|
| <u>Exp. 1</u><br>number of small wells tested | 320 | 320 |
| number of small wells in which partial coloration was detected | 9 | 20 |
| <u>Exp. 2</u><br>number of small wells tested | 320 | 320 |
| number of small wells in which partial coloration was detected | 16 | 51 |

**Claims**

1.  Method for quantitatively and/or qualitatively detecting a specific binding substance in blood or a derived fraction thereof, using the binding affinity between the specific binding substance and its coreactant, by which a reaction component containing peroxidase activity is applied, by incubating a reaction mixture containing the specific binding substance and its coreactant and determining the peroxidase activity in said reaction mixture or a fraction derived therefrom, the peroxidase activity providing a measure of the quantity and/or presence of the specific binding substance, characterized in that the determination of the peroxidase activity is carried out in the presence of a quaternary ammonium salt.

2.  Method for quantitatively and/or qualitatively detecting a specific binding substance in blood or a derived fraction thereof according to Claim 1, characterized in that the quaternary ammonium salt is added together with the substrate.

3.  Method for quantitatively and/or qualitatively detecting a specific binding substance in blood or a derived fraction thereof according to Claim 1, characterized in that the quaternary ammonium salt is added together with the chromogen.

4.  Method for quantitatively and/or qualitatively detecting a specific binding substance in blood or a derived fraction thereof according to Claim 1, characterized in that the quaternary ammonium salt is added together with a substrate/chromogen mixture.

5.  Method for quantitatively and/or qualitatively detecting a specific binding substance in blood or a derived fraction thereof according to Claims 1-4, characterized in that, as quaternary ammonium salt

4

dodecyltrimethylammonium bromide, hexadecyltrimethylammonium bromide or alkyldimethylbenzylammonium chloride is used.

6. Test kit for carrying out the method according to Claim 1, characterized in that the test kit contains:
   - an enzyme conjugate having peroxidase activity,
   - substrate for the enzyme,
   - chromogen, and
   - a quaternary ammonium salt.

**Patentansprüche**

1. Verfahren zum quantitativen und/oder qualitativen Nachweis einer spezifisch bindenden Substanz in Blut oder einer davon abgeleiteten Fraktion unter Anwendung der Bindungsaffinität zwischen der spezifisch bindenden Substanz und ihrem Reaktionspartner, wobei eine Peroxidaseaktivität aufweisende Reaktionskomponente aufgebracht wird, ein Reaktionsgemisch, welches die spezifisch bindende Substanz und ihren Reaktionspartner enthält, inkubiert wird und die Peroxidaseaktivität in diesem Reaktionsgemisch oder einer davon abgeleiteten Fraktion bestimmt wird, wobei die Peroxidaseaktivität ein Mass für die Menge und/oder die Gegenwart der spezifisch bindenden Substanz liefert, dadurch gekennzeichnet, dass die Bestimmung der Peroxidaseaktivität in Gegenwart eines quaternären Ammoniumsalzes durchgeführt wird.

2. Verfahren zum quantitativen und/oder qualitativen Nachweis einer spezifisch bindenden Substanz in Blut oder einer davon abgeleiteten Fraktion nach Anspruch 1, dadurch gekennzeichnet, dass das quaternäre Amminiumsalz zusammen mit dem Substrat zugesetzt wird.

3. Verfahren zum quantitativen und/oder qualitativen Nachweis einer spezifisch bindenden Substanz in Blut oder einer davon abgeleiteten Fraktion nach Anspruch 1, dadurch gekennzeichnet, dass das quaternäre Ammoniumsalz zusammen mit dem Chromogen zugesetzt wird.

4. Verfahren zum quantitativen und/oder qualitativen Nachweis einer spezifisch bindenden Substanz in Blut oder einer davon abgeleiteten Fraktion nach Anspruch 1, dadurch gekennzeichnet, dass das quaternäre Ammoniumsalz zusammen mit einem Substrat/Chromogengemisch zugesetzt wird.

5. Verfahren zum quantitativen und/oder qualitativen Nachweis einer spezifisch bindenden Substanz in Blut oder einer davon abgeleiteten Fraktion nach den Ansprüchen 1 bis 4, dadurch gekennzeichnet, dass als quaternäres Ammoniumsalz Dodecyltrimethylammoniumbromid, Hexadecyltrimethylammoniumbromid oder Alkyldimethylbenzylammoniumchlorid verwendet wird.

6. Testgarnitur zur Durchführung des Verfahrens nach Anspruch 1, dadurch gekennzeichnet, dass die Garnitur enthält:
   - ein Enzymkonjugat, welches Peroxidaseaktivität aufweist,
   - Substrat für das Enzym,
   - Chromogen und
   - ein quaternäres Ammoniumsalz.

**Revendications**

1. Procédé de détection quantitative et/ou qualitative d'une substance de liaison spécifique dans le sang ou dans une fraction qui en dérive en utilisant l'affinité de liaison entre la substance de liaison spécifique et son co-réactif, dans lequel on applique un composant de réaction comprenant une activité de peroxydase, par incubation d'un mélange réactionnel contenant la substance de liaison spécifique et son co-réactif, et on détermine l'activité de peroxydase dans ce mélange réactionnel ou dans une fraction qui en dérive, l'activité de peroxydase procurant une mesure de la quantité et/ou de la présence de la substance de liaison spécifique, caractérisé en ce que la détermination de l'activité de peroxydase est mise en oeuvre en présence d'un sel d'ammonium quaternaire.

2. Procédé de détection quantitative et/ou qualitative d'une substance de liaison spécifique dans le sang ou dans une fraction qui en dérive selon la revendication 1, caractérisé en ce que le sel d'ammonium quaternaire est ajouté en même temps que le substrat.

3. Procédé de détection quantitative et/ou qualitative d'une substance de liaison spécifique dans le sang ou dans une fraction qui en dérive selon la revendication 1, caractérisé en ce que le sel d'ammonium quaternaire est ajouté en même temps que le chromogène.

4. Procédé de détection quantitative et/ou qualitative d'une substance de liaison spécifique dans le sang ou dans une fraction qui en dérive selon la revendication 1, caractérisé en ce que le sel d'ammonium quaternaire est ajouté en même temps qu'un mélange substrat/chromogène.

5. Procédé de détection quantitative et/ou qualitative d'une substance de liaison spécifique dans le sang ou dans une fraction qui en dérive selon les revendications 1 à 4, caractérisé en ce que, comme sel d'ammonium quaternaire, on utilise bromure de dodécyltriméthylammonium, bromure d'hexadécyltri-méthylammoniumou chlorure d'alkyldiméthylbenzylammonium.

6. Trousse ou kit de tests pour la mise en oeuvre du procédé selon la revendication 1, caractérisé en ce que ce kit ou trousse de tests contient:
   - un conjugué d'enzyme exerçant une activité peroxydase,
   - un substrat pour l'enzyme,
   - un chromogène, et
   - un sel d'ammonium quaternaire.